# EUROPÄISCHE PATENTSCHRIFT

(11) **EP 0 830 599 B1**
(45) Veröffentlichungstag und Bekanntmachung des Hinweises auf die Patenterteilung: **05.04.2000**
(21) Anmeldenummer: 96901292.1
(22) Anmeldetag: 17.01.1996
(51) Int. Cl.: G01N 33/50, C12N 1/04

(54) **TESTSYSTEM ZUR ERFASSUNG DER AKTIVITÄT VON NK-ZELLEN**
TEST SYSTEM FOR DETERMINING THE ACTIVITY OF NATURAL KILLER CELLS
SYSTEME TEST SERVANT A DETERMINER L'ACTIVITE DE CELLULES NK

(30) Priorität: 07.06.1995 DE 19520729
(43) Veröffentlichungstag der Anmeldung: 25.03.1998
(73) Patentinhaber: Orpegen Pharma GmbH, 69115 Heidelberg (DE)
(72) Erfinder: HIRT, Werner, D-69115 Heidelberg (DE); EHEMANN, Volker, D-69190 Walldorf (DE); WINK, Alexander, D-69126 Heidelberg (DE)
(74) Vertreter: Weiss, Wolfgang, Dipl.-Chem. Dr.
(86) Internationale Anmeldenummer: EP9600186
(87) Internationale Veröffentlichungsnummer: WO9641163

(56) Entgegenhaltungen:
- WO-A-90/09432
- JOURNAL OF THW NATIONAL CANCER INSTITUTE, Bd. 58, Nr. 3, 1.März 1977, BETHESDA MD USA, Seiten 611-622, XP000569431 H.T. HOLDEN ET AL.: "Standardization of the chromium-51 release, cell-mediated cytotoxicity assay: cryopreservation of mouse effector and target cells. "

## Beschreibung

Die vorliegende Erfindung betrifft ein Verfahren zur Bestimmung der Wirkung von Effektoren auf Targetzellen, insbesondere der Aktivität von natürlichen Killerzellen (NK-Zellen) sowie ein dafür geeignetes Reagenzienkit.

NK-Zellen haben eine wichtige Rolle im Immunsystem von Säugetieren und werden über ihre Funktion definiert als Immunzellen, die eine spontane, nicht MHC-restringierte cytotoxische Aktivität gegen eine Vielzahl von Targetzellen, z. B. Tumorzellen, virusinfizierten Zellen oder allogenen Zielzellen zeigen. Diese Effektoren der natürlichen zellvermittelten Immunität sind morphologisch mehrheitlich große granulierte Lymphozyten und werden immunphänotypisch definiert als CD3 negative Lymphozyten, die CD56 und/oder CD16 exprimieren. Ein kleiner Anteil an T-Lymphozyten (CD3⁺CD56⁺) zeigt ebenfalls NK-Aktivität.

Eine verminderte NK-Aktivität wurde festgestellt bei Patienten mit AIDS, "AIDS related complex" (ARC) [Bonavida, B. et al. 1986, J. Immunol. 137: 1157-1163, Brenner B. et al. 1989, J. Leuk. Biol. 46: 75-83, Quan, C. et al. 1990, J. Acq. Immune Deficiency Syndromes 3: 669-676], Patienten mit Down's Syndrome [Cossarizza, A. et al. 1991, Blood 77: 1263-1270] sowie bei Patienten mit Chronique Fatigue Syndrom [Caligiurri, M. et al. 1987, J. Immunol. 139: 3306-3313, Klimas, N. et al. 1990, J. Clin. Microbiol. 28: 1403-1410, Landay, A. et al. 1991, Lancet 338: 707-713]. Weiterhin ist die NK-Zellaktivität im Verlaufe einer Virusinfektion erhöht [Trinchieri, G. 1989, Adv. Immunol. 47: 187-376]. Patienten im fortgeschrittenen Krebsstadium zeigen gewöhnlich eine verminderte NK-Zellaktivität [Kadish, A.S. et al. 1981, J. Immunol. 127: 1817, Takasugi, M. et al. 1977, Cancer Res. 37: 413] .

Der bislang häufigste Assay zur Bestimmung der cytotoxischen Aktivität von NK-Zellen ist der ⁵¹Cr-Release-Assay [Brunner, K.T. et al. 1968, Immunology 14: 18]. Diese Methode hat allerdings mehrere entscheidende Nachteile für die breite Anwendung in der Routinediagnostik und zwar die Notwendigkeit eines speziell eingerichteten Isotopenlabors, die Verwendung eines radioaktiven Isotops mit einer relativ kurzen Halbwertszeit, hohe Kosten, eine relativ hohe spontane Freisetzung von ⁵¹Cr, sowie lange Markierungs- und Inkubationszeiten.

Untersuchungen zur Standardisierung des ⁵¹Cr-Release-Assays unter Verwendung einer Kryokonservierung von Effektor- und Targetzellen werden in J. Natl. Cancer Inst., 58 (3) (1977), Seiten 611-622 beschrieben. Dabei werden unmarkierte Targetzellen eingefroren und nach dem Auftauen mit ⁵¹Cr markiert. Die aufgetauten Zellen können also nicht unmittelbar im Assay verwendet werden, sondern müssen erst einer Vorbehandlung unterzogen werden.

Andere Methoden zur Quantifizierung der Cytotoxizität sind beschrieben worden und beinhalten die Messung von freigesetzten Substanzen wie z.B. Fluoreszenzfarbstoffen (Europium [Blomberg, K. et al. 1986, J. Immunol. Methods 86: 225] oder H33342 [Brenan, M. 1988, J. Immunol. Methods 112: 121]) oder die Bestimmung der Enzymaktivität von freigesetzten cytoplasmatischen Enzymen [Korzeniewski, C. 1983, J. Immunol. 64: 313].

WO90/09432 beschreibt Fluorophor-markierte kryokonservierte Zellen für die Verwendung als Targetzellen in Zytotoxizitätsassays. Es werden nach dem Auftauen jedoch nur Zellen mit einer relativ geringen Vitalitätsrate von < 85 % erhalten, so daß Wechselwirkungen zwischen Effektoren und toten Targetzellen die Ergebnisse negativ beeinflussen können.

Weiterhin sind durchflußzytometrische Methoden zur Bestimmung der NK-Effektoraktivität beschrieben, die es ermöglichen, die Zytotoxizität auf einer Einzelzellebene zu beobachten [McGinnes, K. et al. 1986. J. Immunol. Methods 86: 7-15, Zarzone, D. et al. 1986, J. Immunol. Methods 94: 247-255, Papa, S. et al. 1988, J. Immunol. Methods 107: 73-78]. Diese Methoden haben allerdings den Nachteil, daß nicht eindeutig zwischen Ziel- und Effektorzellen unterschieden werden kann. Daher sind durchflußzytometrische Methoden entwickelt worden, bei denen die Membranen der Targetzellen mit fluoreszenten Farbstoffen zur Unterscheidung von Effektorzellen markiert werden [Slezak, S. E. et al. 1989, J. Immunol. Methods 117: 205-214, Radosevic, K. et al. 1990, J. Immunol. Methods 135: 81-89].

Kroesen, B.-J. et al. [J. Immunol. Methods 156 (1992): 47-54] und Chang, L. et al. [J. Immunol. Methods 166 (1993): 45-54] beschreiben ein Verfahren zur Bestimmung der NK-Aktivität unter Verwendung des lipophilen Membranfarbstoffs 3,3'-Dioctadecyloxacarbocyanin Perchlorat (DiOC₁₈). Als Targetzellen wurde die in Zellkultur gehaltene NK-sensitive humane erythromyelozytäre Leukämie-Zellinie K562 [Lozzio, B.B. et al. 1976, J. Natl. Cancer Inst. (U.S.) 56: 627] eingesetzt.

Ein Nachteil der bisher beschriebenen Verfahren ist die Notwendigkeit der Anzucht der Targetzellen in einem Zellkulturlabor vor der eigentlichen Durchführung des Assays.

Aufgabe der vorliegenden Erfindung war es daher, ein Verfahren zu entwickeln, bei dem die Nachteile des Standes der Technik mindestens teilweise beseitigt sind. Insbesondere sollte ein Verfahren zur Kryokonservierung von Zellen entwickelt werden, bei dem die Zellen bei -70°C ohne Verlust der Vitalität gelagert werden können und somit ohne vorherige Kultivierung direkt in einen diagnostischen Test, den durchflußzytometrischen NK-Test, z.B. eingesetzt werden können.

Diese Aufgabe wird gelöst durch ein Verfahren zur Bestimmung der Wirkung von Effektoren auf Targetzellen, welches dadurch gekennzeichnet ist, daß man als Targetzellen aufgetaute kryokonservierte Säugerzellen ohne vorherige Kultivierung verwendet, die nach Lagerung bei -70°C über einen Zeitraum von 4 Wochen eine Vitalität von mindestens 90 % besitzen und die erhältlich sind durch Herstellung einer Suspension der Targetzellen in einem Medium, Markierung der Targetzellen, Kultivierung der markierten Zellen für 4-6n, Zusatz eines Kryokonservierungsmittels bis zu einer Endkonzentration von 2-10% (v/v) und Einfrieren der Zellen bei einer Temperaturabnahme von nicht mehr als 2°C pro Minute. Das Einfrieren erfolgt vorzugsweise bis zu einer Temperatur von mindestens -50°C, besonders bevorzugt von mindestens -65°C und am meisten bevorzugt von etwa -70°C, um die Targetzellen schonend unter Erhalt ihrer Vitalität zu kryokonservieren. Überraschenderweise wurde gefunden, daß die kryokonservierten Zellen eine ausreichend hohe Vitalität besitzen, um für die Verwendung als Targetzellen in der klinischen Diagnostik geeignet zu sein.

Vorzugsweise verwendet man Targetzellen, die aus einer Zellkultur stammen, insbesondere eine humane Zellinie. Besonders bevorzugt sind humane Tumor- oder Leukämiezellinien. Am meisten bevorzugt werden die Zellinien ausgewählt aus der chronischen myelogenen Leukämie-Zellinie K562 (ATCC CCL 243), der akuten lymphoblastischen Leukämie-T-Zellinie MOLT-4, der akuten promyelocytären Leukämie-Zellinie HL-60, der Ovarialcarcinom-Zellinie Goodwin und der Alveolarzellcarcinom-Zellinie A 549. Bezüglich dieser Zellinien und ihrer Verwendung als Targetzellen in NK-Assays wird auf Pross et al. (J. Clin. Immunol. 1 (1981), 51-63) und die dort zitierten Literaturstellen verwiesen.

Vorzugsweise werden Zellen aus einer Zellkultur in exponentieller Wachstumsphase verwendet. Nach Ernten werden diese Zellen auf eine Konzentration von 1x 10⁵ bis 5x 10⁶/ml, besonders bevorzugt etwa 1x 10⁶/ml eingestellt, durch Kühlung auf eine Temperatur von 0 - 4 °C gebracht und mit dem Kryokonservierungsmittel versetzt. Die Endkonzentration des Kryokonservierungsmittels im Medium ist vorzugsweise 4 - 8 % (v/v), besonders bevorzugt etwa 5 %. Als Kryokonservierungsmittel verwendet man vorzugsweise Dimethylsulfoxid (DMSO) oder Glycerin. Besonders bevorzugt verwendet man DMSO.

Das Einfrieren der Zellen erfolgt bei einer Temperaturabnahme von nicht mehr als 2 °C pro Minute, vorzugsweise bei einer Temperaturabnahme von 0,5-1,5 °C pro Minute und besonders bevorzugt bei einer Temperaturabnahme von etwa 1 °C pro Minute.

Durch das erfindungsgemäße Verfahren sind kryokonservierte Säugetierzellen erhältlich, die nach Lagerung über einen Zeitraum von 4 Wochen und vorzugsweise von 12 Wochen noch eine Vitalität von mindestens 90 % besitzen. Eine Vitalität von mindestens 90 % bedeutet in diesem Zusammenhang, daß nach dem Einfrieren und Auftauen noch mindestens 90 % aller vor dem Einfrieren lebensfähigen Zellen noch lebensfähig sind. Die Vitalität der Zellen kann durch bekannte Methoden, z.B. einen Farbausschlußtest mit Trypanblau oder Erythrocin B bestimmt werden. Bei diesem Farbausschlußtest wird eine Farbstofflösung den Zellen zugesetzt und die Anzahl der toten Zellen, die den Farbstoff nicht aufnehmen können, gegenüber der Gesamtzahl der getesteten Zellen bestimmt.

Das erfindungsgemäße Verfahren betrifft eine Bestimmung der Wirkung von Effektoren auf Targetzellen. Die Effektoren können chemische oder biologische Substanzen, Zellen, Mikroorganismen oder Kombinationen davon sein. Vorzugsweise werden als Effektoren Zellen, insbesondere Natürliche Killerzellen verwendet.

Ein wichtiger Punkt des erfindungsgemäßen Verfahrens ist, daß der Test ohne vorherige Kultivierung der Targetzellen durchgeführt werden kann, die Targetzellen können unmittelbar vor Durchführung des Assays aufgetaut werden. Dies bedeutet, daß die Targetzellen innerhalb eines Zeitraums von vorzugsweise maximal 8 h, besonders bevorzugt von maximal 2 h und am meisten bevorzugt von maximal 1 h nach dem Auftauen in den Assay eingesetzt werden. Der Assay kann so mit standardisierten Targetzellen durchgeführt werden. Dies ist eine Voraussetzung für den Einsatz in der Routinediagnostik. Daher war es notwendig ein Verfahren zu etablieren, welches die Zellen möglichst schonend unter Erhalt der Vitalität kryokonserviert. Hierzu werden für einen NK-Test beispielsweise K562 Zellen in der Log-Phase geerntet, auf eine Konzentration von 1 x 10⁶ ml eingestellt und mit DMSO versetzt ad 5 % (v/v). Das Einfrieren der Zellen erfolgt bis -70 °C mit einer Temperaturabnahme von 1 °C pro Minute. Die Zellen werden bei -70°C in einem Tiefkühlschrank gelagert.

Zur Unterscheidung der Effektorzellen von den Targetzellen werden die Targetzellen markiert. Es wurde überraschenderweise festgestellt, daß eine Markierung der Targetzellen bereits vor der Kryokonservierung möglich ist. Diese Vorgehensweise hat den Vorteil, daß die bereits markierten Targetzellen nach dem Auftauen direkt in den Assay eingesetzt werden können.

Gemäß einer bevorzugten Ausführungsform werden die Targetzellen vor der Kryokonservierung mit einer ersten Markierungssubstanz markiert, nach dem Auftauen ohne vorherige Kultivierung mit einer NK-Zellen enthaltenden Probe gemischt und inkubiert, die abgetöteten Targetzellen mit einer zweiten Markierungssubstanz spezifisch markiert und die Markierung bestimmt.

Die erste und zweite Markierungssubstanz werden so gewählt, daß sie nebeneinander nachweisbar sind.

Als erste Markierungssubstanz zur Markierung der Targetzellen wird vorzugsweise ein Membranfarbstoff eingesetzt. Vorzugsweise verwendet man einen lipophilen Fluoreszenzfarbstoff, z.B. 3,3'-Dioctadecyloxacarbocyanin Perchlorat (DiOC₁₈) [Badley, R.A. et al. 1973. Biochemistry 12: 268]. Die Verwendung von DiOC₁₈ zur Markierung von K562 Targetzellen im NK-Assay wurde bereits beschrieben [Kroesen, B.-J, et al. J. Immunol. Methods 156 (1992): 47-54; Chang, L. et al, J. Immunol. Methods 166 (1993): 45-54].

Der Membranfarbstoff DiOC₁₈ wird vorzugsweise in einer Konzentration von 0,5 - 2 mg/ml, insbesondere ca. 1,25 mg/ml (1,42 mM) in Dimethylsulfoxid gelöst. Für die Färbung werden von dieser Stammlösung 20 µl zu ca. 1 x 10⁶ Targetzellen in 5 ml Komplettmedium zugegeben. Daraus resultiert eine Endkonzentration von 4,85 µg/ml und eine DMSO-Konzentration von 0,4 % (v/v). Damit wird gleichzeitig eine zu hohe und für Zellen toxische Konzentration an organischem Lösungsmittel vermieden. Die Zellen werden zur Färbung vorzugsweise 5 - 30 min., z.B. 15 min. mit dem Membranfarbstoff bei 37°C inkubiert und anschließend einmal mit Komplettmedium gewaschen (120 g, 3 min.).

Mononukleäre Effektorzellen werden durch Standardverfahren (Dichtegradientenzentrifugation über Ficoll oder mittels Leuko-PREP Zellseparationsröhrchen 13 mm bzw. 16 mm (Becton Dickinson), aus Blut, z.B. heparinisiertem oder Citrat-Vollblut gewonnen. Die Anwesenheit von Monozyten in den Testansätzen kann die zytotoxische Aktivität der NK-Zellen vermindern. Die NK-Aktivität der isolierten Lymphozyten kann somit durch Depletion der Monozyten erhöht werden. Nichtadhärente Effektorzellen lassen sich z.B. durch einen Plastikadhärenzschritt oder Passage über Nylonwolle anreichern.

In einer bevorzugten Ausführungsform des erfindungsgemäßen Verfahrens führt man jeweils Doppelbestimmungen mit unterschiedlichen Verhältnissen von Effektoren zu Targetzellen durch. Vorzugsweise verwendet man die Verhältnisse 50 : 1, 25 : 1 und 12,5 : 1. Um einen engen Kontakt zwischen Effektor- und Zielzellen herzustellen, werden die Zellsuspensionen kurz anzentrifugiert (100 g, 1 min). Die Inkubationszeit der Effektorzellen mit den Targetzellen kann 30 bis 240 min, vorzugsweise 120 min betragen. Es ist aber auch möglich, das erfindungsgemäße Verfahren in mehreren parallelen Ansätzen mit verschiedenen Inkubationszeiten durchzuführen.

Die NK-Effektoraktivität kann durch Immunmodulatoren, z.B. Interleukin-2 oder Interferon-alpha, gesteigert werden. In einer bevorzugten Ausführungsform des erfindungsgemäßen Verfahren führt man zwei parallele Ansätze durch, von denen ein Ansatz zusätzlich Interleukin-2 in einer Konzentration von 30 U/ml enthält.

Die toten Targetzellen werden durch Zugabe einer zweiten Markierung, vorzugsweise eines DNA-Farbstoffs, z.b. eines fluoreszierenden DNA-Farbstoffs angefärbt. Als DNA-Farbstoff kann ein zu diesem Zweck bekannter und geeigneter Farbstoff verwendet werden, der eine andere Emissionswellenlänge hat als der für die Membranmarkierung der Targetzellen verwendete erste Farbstoff. Vorzugsweise verwendet man Propidiumjodid als DNA-Farbstoff.

Der letzte Schritt des erfindungsgemäßen Verfahrens ist die Bestimmung der Markierung, z.B. der Fluoreszenz. Die Auswertung kann dabei mittels Durchflußzytometer oder Mikroskop erfolgen.

Weiterhin ein Gegenstand der vorliegenden Erfindung ist ein Reagenzienkit, insbesondere zur Bestimmung der NK-Effektoraktivität von humanen peripheren Lymphozyten. In einer Ausführungsform enthält das Kit mit einem Membranfarbstoff markierte kryokonservierte Targetzellen und einen DNA-Farbstoff physikalisch voneinander getrennt. Vorzugsweise enthält das Reagenzienkit außerdem ein Medium für die Zellkultur oder/und einen Modulator für NK-Zellen, z.B. ein Stimulans wie etwa Interleukin-2. Die Bestandteile des Reagenzienkits können sowohl fertige Lösungen sein oder auch Lyophilisate darstellen, aus denen der Anwender die Lösungen erst erstellt.

In einer besonders bevorzugten Ausgestaltung der obigen Ausführungsform enthält das Kit anstelle der obigen Komponenten 1 und 2 kryokonservierte Targetzellen, die bereits mit DiOC₁₈ markiert sind.

Als Meßgerät wird vorzugsweise ein Durchflußzytometer (z.B. FACScan, Fa. Becton Dickinson) mit 488 nm Lichtanregung (Argonlaser) verwendet.

Die Komponenten des Reagenzienkits werden im Dunkeln bei unterschiedlichen Temperaturen aufbewahrt:
- kryokonservierte Targetzellen bei -70°C
- Membranfarbstoff und Interleukin-2 bei -20°C
- Komplettmedium und DNA-Färbelösung bei 4°C

Die Erfindung wird durch die folgenden Beispiele und Abbildungen 1-11 erläutert.

Es zeigen:
- Abb. 1: ein durchflußzytometrisches Streulichtdiagramm der Targetzellen K562,
- Abb. 2: das Setzen eines "Live Gate" für Farbstoff-markierte Targetzellen,
- Abb. 3 und 4: durchflußzytometrische Fluoreszenzdiagramme von Targetzellen nach einer Inkubationszeit von 60 bzw. 120 min. mit Effektoren,
- Abb. 5: ein Fluoreszenzhistogramm der Targetzellen ohne Effektoren,
- Abb. 6: ein Fluoreszenzhistogramm der Targetzellen nach Inkubation mit Effektoren,
- Abb. 7: ein Fluoreszenzhistogramm der Targetzellen nach Inkubation mit durch Interleukin-2 stimulierten Effektoren,
- Abb. 8: ein Diagramm, das die Vitalität von DiOC₁₈-markierten K562-Targetzellen in Abhängigkeit von der Kryokonservierungsdauer zeigt,
- Abb. 9: ein Diagramm, das die Fluoreszenzintensität von DiOC₁₈-markierten K562-Targetzellen in Abhängigkeit von der Kryokonservierungsdauer zeigt,
- Abb. 10: den Vergleich von NK-Assays mit markiert und unmarkiert eingefrorenen K562 Targetzellen und
- Abb. 11: den Vergleich eines erfindungsgemäßen NK-Assays mit dem klassischen ⁵¹Cr-Release-Assay.

### Beispiel 1

### Anzucht und Krykonservierung der Targetzellen K562

Material und Geräte:
1. K562 Targetzellinie (ATCC, Rockville, MD, No. CCL 243)
2. RPMI 1640-Zellkulturmedium (Fa. Cytogen)
3. Fötales Kälberserum (FCS) (Fa. Sigma)
4. Zellkulturflaschen 225 cm² (Fa. Costar)
5. Dimethylsulfoxid p.a. > 99,5 % (Fa. Fluka)
6. Einfrierröhrchen (Fa. Costar)
7. Laminar-Flow-Reinraumwerkbank (Fa. Dan VFR 1206)
8. Zentrifuge (Fa. Hermle Z364)
9. 50 ml-Einmal-Zentrifugenröhrchen (Fa. Costar)

K562 Zellen werden in RPMI 1640-Zellkulturmedium mit 10 % (v/v) FCS (Komplettmedium) in Standard-Zellkulturflaschen bei 37°C und 5% CO₂ im Brutschrank kultiviert. Die Zellen werden nach 48 h Kulturdauer passagiert. Die "Doubling Time" der K562 Zellinie beträgt 18 h.

Die Einfrierbedingungen wurden für diese Zellinie und die Verwendung der Zellen im NK-Test entwickelt und validiert. Zellen in der exponentiellen Wachstumsphase werden geerntet und durch Zentrifugation (600 rpm, 8 Minuten) pellettiert. Das Zellpellet wird in frischem Komplettmedium aufgenommen. Dann wird die Zahl der Zellen und deren Vitalität bestimmt. Die Zellsuspension wird anschließend in ein Eiswasserbad gestellt, ad 5 % /v/v) mit Dimethylsulfoxid versetzt und kurz gut gemischt. Die Zellen werden mit einer Vitalität > 98 % in einer Zellkonzentration von 1 x 10⁶ Zellen pro ml in Einfrierröhrchen aliquotiert. Das Einfrieren der Zellen erfolgt bis -70 °C mit einer Temperaturabnahme von 1 °C pro Minute. Die Zellen werden bei -70 °C in einem Tiefkühlschrank gelagert.

Die bei -70°C gelagerten Zellen behalten ihre hohe Vitalität (> 90 % vitale Zellen) über einen Zeitraum von mindestens 12 Wochen. Die Vitalitätsbestimmung erfolgte mit der Trypanblaumethode. Die Ergebnisse von 3 verschiedenen Einfrierexperimenten sind in Tabelle 1 wiedergegeben.

**Tabelle 1**

| Wochen nach Einfrieren der Zellen | Vitalität (%) Exp. 1 | Vitalität (%) Exp. 2 | Vitalität Exp. 3 |
|---|---|---|---|
| Vor Einfrieren | 98 | 99 | 99 |
| 1 | 97 | 98 | 99 |
| 2 | 97 | 98 | 99 |
| 3 | 95 | 98 | 97 |
| 4 | 95 | 97 | 97 |
| 5 | | 98 | 96 |
| 6 | 96 | 97 | 96 |
| 7 | 96 | 97 | 96 |
| 8 | 94 | 96 | |
| 9 | 95 | 96 | |
| 10 | 93 | | |
| 11 | | | 93 |
| 12 | | | 94 |
| 13 | | 95 | |
| 14 | | 95 | |
| 16 | | 95 | 93 |
| 18 | | 94 | |

### Beispiel 2:

### Bestimmung der NK-Aktivität von humanen peripheren Blutlymphozyten (PBLs)

Material und Geräte
1. Kühlzentrifuge mit freischwingenden Einsätzen (z.B. Hermle Zentrifuge ZK 364)
2. Wasserbad (Fa. GFL, Typ 1083)
3. Laminar-Flow-Reinraumwerkbank
4. Vortex-Mischer (Vortex-Genie, Modell K-550-GE)
5. Neubauer-Zählkammer mit Deckglas
6. Variable Transferpipetten
7. Durchflußzytometer mit einer Anregungswellenlänge von 488 nm (z.B. FACScan, Becton Dickinson)
8. 5 ml Falcon Probenröhrchen (12 x 75 mm, Becton Dickinson) mit Stopfen
9. EDTA (K₃) Blutentnahme-Röhrchen (z.B. Becton Dickinson)
10. LeucoPrep 13 bzw. 16 mm Zelltrenn-Röhrchen (Becton Dickinson) oder Isopaque-Ficoll (Histopaque®, Sigma, oder Ficoll-Paque®, Pharmacia) zur Präparation der mononukleären Zellen
11. 15 ml-Einmal-Zentrifugenröhrchen (z.B. Falcon oder Costar)
12. 50 ml-Einmal-Zentrifugenröhrchen (z.B. Costar)
13. Steriles PBS oder physiologische Kochsalzlösung
14. Eisbad mit Deckel
15. Pipettenspitzen
16. Kryokonservierte Targetzellen K562, wie in Beispiel 1 beschrieben
17. RPMI 1640-Zellkulturmedium (Fa. Cytogen)
18. Fötales Kälberserum (FCS) (Fa. Sigma)
19. Propidiumjodid (Fa. Sigma)
20. Membranfarbstoff DiOC₁₈ (Fa. Molecular Probes)

Effektorzellen werden aus 5-10 ml Citrat-Blut, EDTA-Blut oder Heparin-Blut durch Dichtegradientenzentrifugation (LeukoPREP-Zellseparationsröhrchen oder Ficoll-Hypaque) gewonnen. Die isolierten mononukleären Zellen werden anschließend auf eine Zellkonzentration von 5 x 10⁶ Zellen/ml Komplettmedium eingestellt.

In ein 50 ml Zentrifugenröhrchen werden ca. 50 ml Komplettmedium vorgelegt und im Wasserbad auf 37°C vorgewärmt. Die bei -70°C gelagerten K562 Zellen werden schnell, d.h. innerhalb von 2 min. im Wasserbad aufgetaut und sofort in das 50 ml Zentrifugenröhrchen mit vorgelegtem Medium überführt. Das Röhrchen wird kurz geschwenkt und abzentrifugiert (120 x g, 2-3 min). Die Zellen werden einmal mit 15 ml Komplettmedium gewaschen (120 x g, 2-3 min).

Anschließend wird das Medium entfernt, die Zellen werden in 5 ml Komplettmedium resuspendiert. Nach Zugabe von 20 µl des Membranfarbstoffs DiOC₁₈ (1,4 mM) und sofortigem kurzem Vortexen wird die Zellsuspension 15 min bei 37°C im Wasserbad inkubiert. Anschließend werden 10 ml Komplettmedium zugegeben und abzentrifugiert (120 x g, 2-3 min). Die Zellen werden in 1 ml Medium aufgenommen und auf eine Zellkonzentration von 1 x 10⁵/ml eingestellt.

Je nach gewünschtem Verhältnis von Effektoren zu Targetzellen werden folgende Zellmischungen durch Zusammenpipettieren in ein Zentrifugenröhrchen mit Schnappdeckel hergestellt.

| Verhältnis E:T | Effektoren (µl) | Targetzellen (µl) | Komplettmedium |
|---|---|---|---|
| 50 : 1 | 100 | 100 | 0 |
| 25 : 1 | 50 | 100 | 50 |
| 12,5 : 1 | 25 | 100 | 75 |

Es empfiehlt sich jeweils Doppelbestimmungen durchzuführen. Als Kontrolle dient ein Ansatz ohne Zugabe von Effektoren, um die spontane Absterberate der Targetzellen zu bestimmen. Als Positivkontrolle dienen Ansätze, denen je 30 µl Interleukin-2 (= 30 U/ml, 6 U/Ansatz) zugegeben worden sind, wobei die Effektorzellen hierfür nicht mit IL-2 vorinkubiert werden müssen.

Die Zentrifugenröhrchen werden kurz anzentrifugiert (100 g, 1 min). Die Inkubation der Ansätze erfolgt im Wasserbad bei 37°C in Zentrifugenröhrchen mit aufgesetztem Schnappdeckel. Nach der Inkubationszeit von 30, 60, 120 oder 240 min werden die Proben ins Eiswasserbad gestellt, mit je 50 µl DNA-Färbelösung versetzt (40 µg/ml Propidiumjodid), durch Vortexen kurz gemischt und nach 10 min. durchflußzytometrisch analysiert.

Um eine deutliche NK-Aktivität feststellen zu können, ist eine Inkubationszeit von 120 min. zu empfehlen.

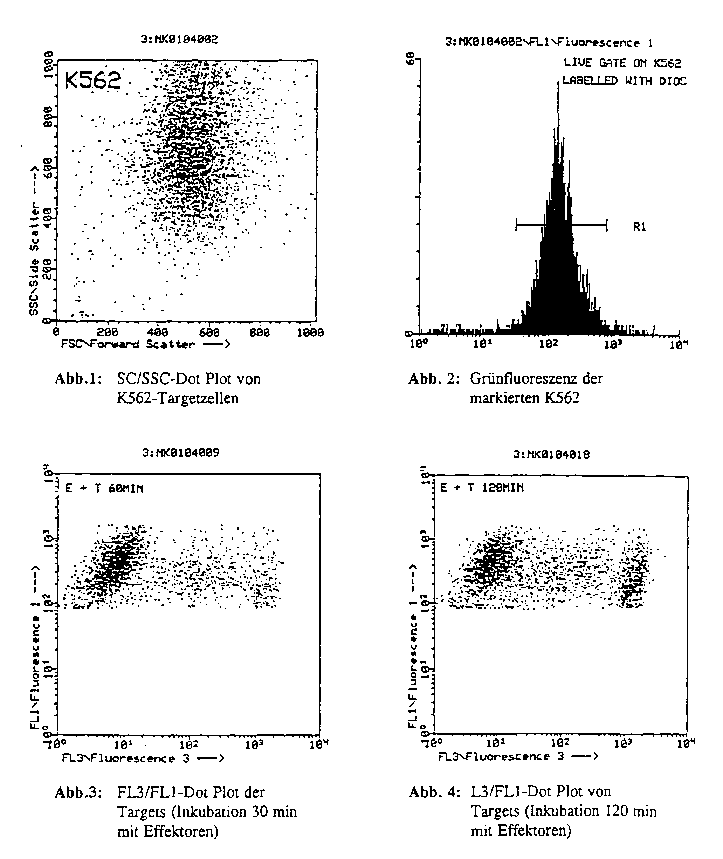

Die Auswertung erfolgt im Durchflußzytometer (z.B. FACScan) unter Verwendung von handelsüblicher Software (z.B. LYSIS II). Die Fluoreszenzsignale werden im Falle der Grünfluoreszenz (DiOC) durch ein 530/30 nm "Bandpass"-Filter, im Falle der Rotfluoreszenz (Propidiumjodid) entweder durch ein 585 nm "Bandpass" oder ein > 650 nm "Longpass"-Filter gesammelt. Die Fluoreszenzparameter werden logarithmisch (4 Dekaden), die Streulichtparameter (Vorwärts- und Seitwärtsstreulicht) linear verstärkt. Das Streulichtdiagramm der Targetzellen K562 ist in Abb. 1 dargestellt.

Für die durchflußzytometrische Analyse der Zytotoxizität ist es essentiell, die Targetzellen von den Effektor-Zellen zu unterscheiden. Zu diesem Zweck werden durch das Setzen eines "Live Gate" nur grünfluoreszente Targetzellen registriert (Abb. 2).

Abb. 3 und 4 zeigen die FL3/FL1-Dot Plot-Analysen der Targetzellen nach einer Inkubationszeit von 60 bzw. 120 min mit Effektoren.

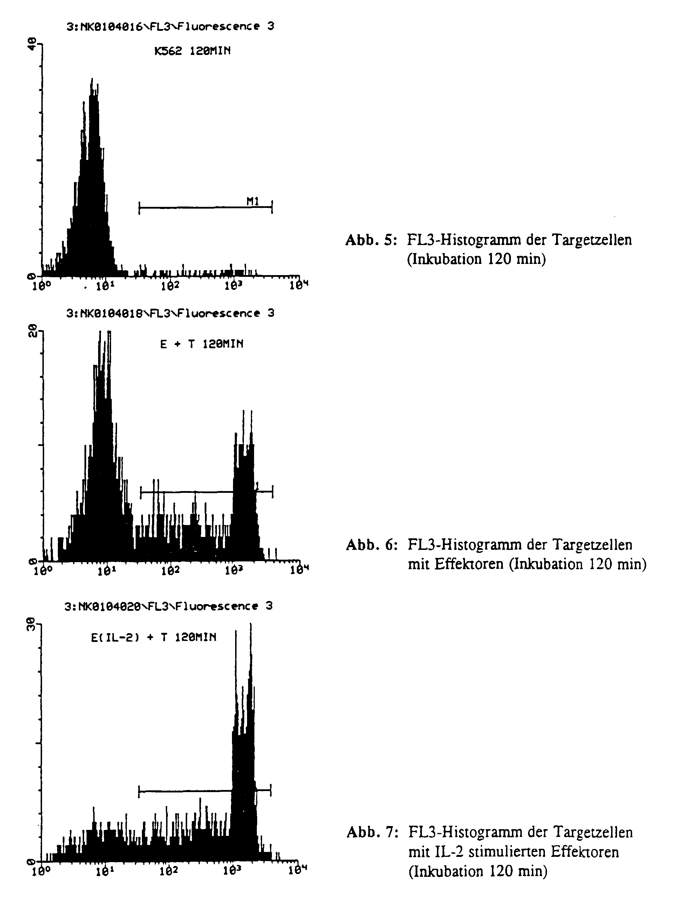

Ausgewertet wird der Anteil an toten, d.h. rotfluoreszenten Targetzellen. Der Prozentsatz an Zytotoxizität wird berechnet durch Subtraktion des Anteils an toten Zellen in der Probe mit Targetzellen allein ohne Effektoren (Abb. 5, im Beispiel 2,4 %) vom Anteil an toten Targetzellen in den Testansätzen mit zugegebenen Effektoren (Abb. 6 im Beispiel 40,9 %). In Abb. 7 ist das Histogramm der FL3-Fluoreszenz von Targetzellen wiedergegeben nach 120minütiger Inkubation mit Effektoren unter Zusatz von 30 U/ml Interleukin-2. Hier lag der Anteil an toten Targetzellen bei 81,5 %.

### Beispiel 3:

### Kryokonservierung von markierten Targetzellen

K562 Targetzellen wurden in der Log-Phase geerntet und auf eine Konzentration von 2 x 10⁶/ml in Komplettmedium eingestellt. Für die Markierung mit dem lipophilen Membranfarbstoff DiOC₁₈ wurden 20 µl der Stammlösung (1,42 nM in DSMO) zu 5 ml der Targetzellsuspension in Komplettmedium gegeben. Die Zellen wurden 15 min. bei 37°C inkubiert, einmal mit Komplettmedium gewaschen (120 g, 3 min.) und anschließend für 4-6 h erneut im Brutschrank inkubiert. Dann wurden die gefärbten Targetzellen, wie für die ungefärbten Zellen beschrieben, eingefroren und bei -70°C unter Erhalt der Vitalität gelagert.

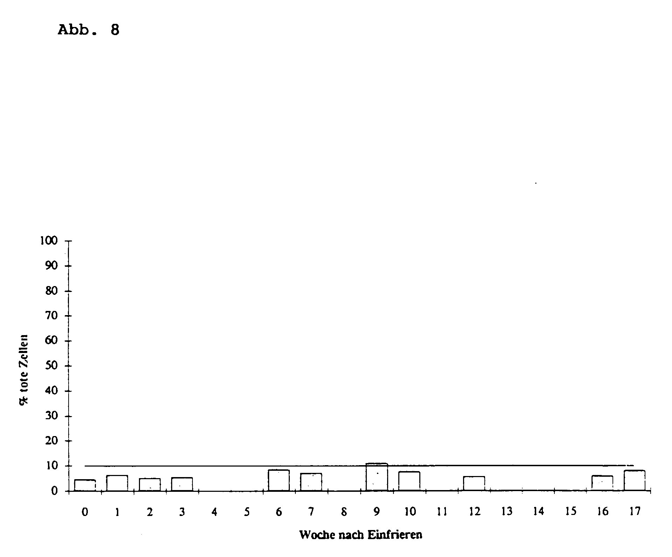

Abbildung 8 zeigt ein Diagramm, in dem die Vitalität von markiert eingefrorenen K562 Targetzellen in Abhängigkeit von der Kryokonservierungsdauer dargestellt ist. Man erkennt, daß selbst nach einer Kryokonservierungsdauer von 17 Wochen die Vitalität überraschenderweise noch >90% ist.

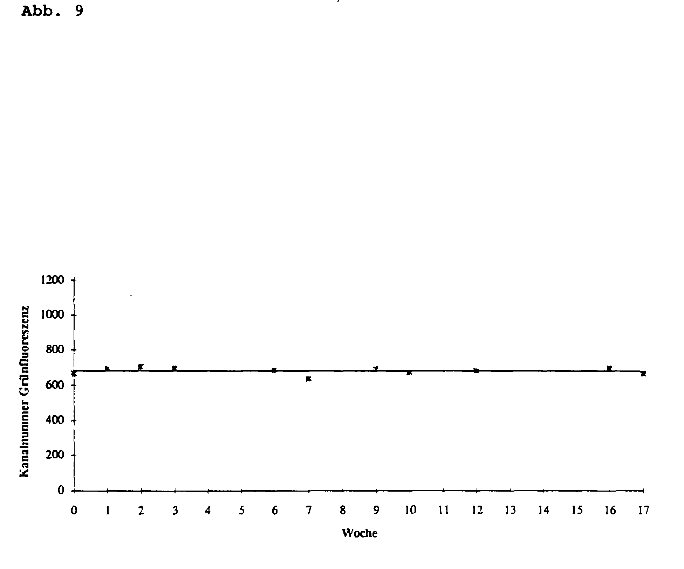

Abbildung 9 zeigt ein Diagramm, das die Fluoreszenzintensität von markiert eingefrorenen K562 Targetzellen in Abhängigkeit von der Kryokonservierungsdauer wiedergibt. Man erkennt, daß die Grünfluoreszenz eine hohe Stabilität aufweist und auch bei längerer Kryokonservierungsdauer keine signifikante Änderung zeigt.

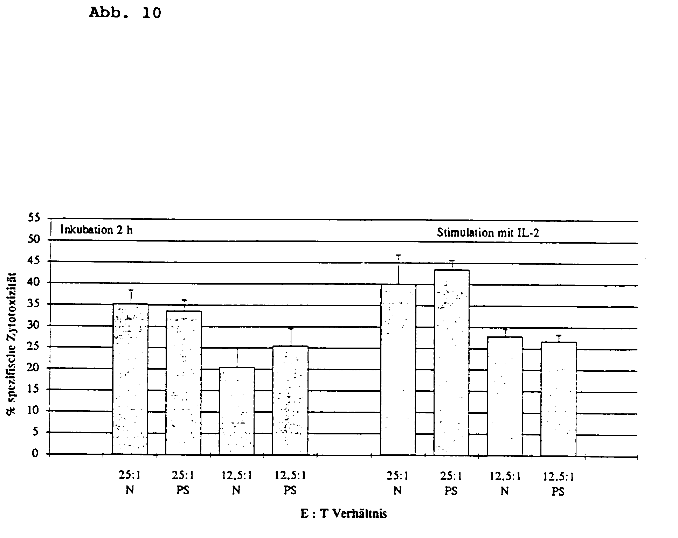

Markiert eingefrorene K562 Targetzellen wurden in einem NK-Assay mit Targetzellen verglichen, die erst nach dem Auftauen mit dem lipophilen Membranfarbstoff DiOC₁₈ markiert worden waren. Dieser Vergleich ist in Abbildung 10 gezeigt. Markiert (PS) und unmarkiert (N) eingefrorene K562 Zellen wurden in einem Verhältnis von Effektorzellen (E) zu Targetzellen (T) von 25 : 1 bzw. 12,5 : 1 eingesetzt. Der Test wurde durch zweistündige Inkubation der Effektor- und Targetzellen ohne bzw. mit Stimulation mit IL-2 durchgeführt. Wie aus Abbildung 10 hervorgeht, ergaben sich keine statistisch signifikanten Unterschiede der markiert und unmarkiert eingefrorenen Targetzellen hinsichtlich der spezifischen Cytotoxizität.

### Beispiel 4:

### Vergleich des erfindungsgemäßen Verfahrens mit dem klassischen ⁵¹Cr-Release-Assay

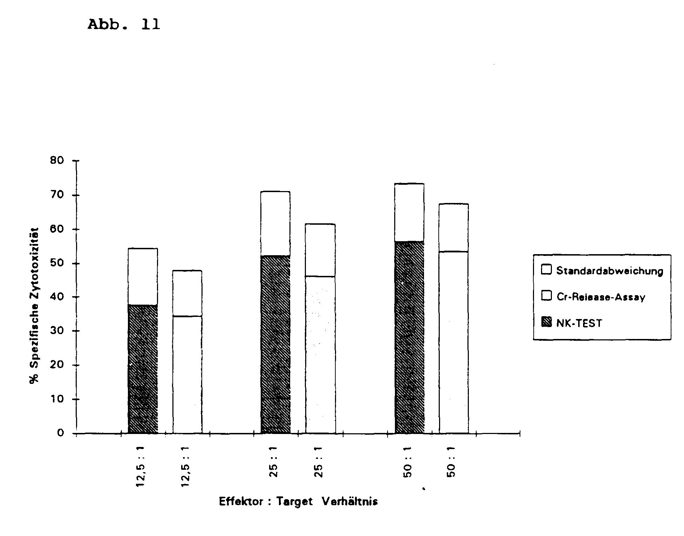

Das erfindungsgemäße Verfahren wurde mit dem klassischen ⁵¹Cr-Release-Assay verglichen. Dazu wurden insgesamt 25 parallele Bestimmungen bei drei verschiedenen Verhältnissen von Effektoren zu Targetzellen (50:1, 25:1 und 12,5:1) durchgeführt. Die Inkubationsdauer betrug jeweils 4 h. Die Ergebnisse dieses Vergleiches sind in der Abbildung 11 gezeigt. Wie aus dieser Abbildung ersichtlich, ist das neue Verfahren dem alten an Präzision gleichwertig, aber wesentlich schneller.

## Patentansprüche

1. Verfahren zur Bestimmung der Wirkung von Effektoren auf Targetzellen,
**dadurch gekennzeichnet,**
daß man als Targetzellen aufgetaute kryokonservierte Säugerzellen ohne vorherige Kultivierung verwendet, die nach Lagerung bei - 70°C über einen Zeitraum von 4 Wochen eine Vitalität von mindestens 90 % besitzen und erhältlich sind durch Herstellung einer Suspension von Targetzellen in einem Medium, Markieren der Targetzellen, Kultivieren der markierten Zellen für 4-6 h, Zusatz eines Kryokonservierungsmittels bis zu einer Endkonzentration von 2-10 % (v/v) und Einfrieren der Zellen mit einer Vitalität > 98% bei einer Temperaturabnahme von nicht mehr als 2°C pro Minute.

2. Verfahren nach Anspruch 1,
**dadurch gekennzeichnet,**
daß man kryokonservierte Zellen verwendet, die nach Lagerung bei -70°C über einen Zeitraum von 12 Wochen eine Vitalität von mindestens 90% besitzen.

3. Verfahren nach Anspruch 1 oder 2,
**dadurch gekennzeichnet,**
daß man als Targetzellen eine humane Tumor- oder Leukämie-zellinie verwendet.

4. Verfahren nach Anspruch 3,
**dadurch gekennzeichnet,**
daß man eine humane Zellinie verwendet, die ausgewählt wird aus der chronischen myelogenen Leukämie-Zellinie K562 (ATCC CCL 243), der akuten lymphoblastischen Leukämie-T-Zellinie MOLT-4, der akuten promyelocytären Leukämie-Zellinie HL-60, der Ovarialcarcinom-Zellinie Goodwin und der Alveolarzellcarcinom-Zellinie A 549.

5. Verfahren nach einem der Ansprüche 1 bis 4,
**dadurch gekennzeichnet,**
daß man die Aktivität von natürlichen Killerzellen (NK-Zellen) bestimmt und die Targetzellen vor der Kryokonservierung mit einer ersten Markierungssubstanz markiert, nach dem Auftauen ohne vorherige Kultivierung mit einer NK-Zellen enthaltenden Probe mischt und inkubiert, die abgetöteten Targetzellen mit einer zweiten Markierungssubstanz spezifisch markiert und die Markierung bestimmt.

6. Verfahren nach einem der Ansprüche 1 bis 5,
**dadurch gekennzeichnet,**
daß man die Targetzellen mit einem Membranfarbstoff, insbesondere mit einem lipophilen Fluoreszenzfarbstoff markiert.

7. Verfahren nach Anspruch 6,
**dadurch gekennzeichnet,**
daß man als Membranfarbstoff 3, 3'-Dioctadecyloxacarbocyanin oder ein Salz davon verwendet.

8. Verfahren nach einem der Ansprüche 5 bis 7,
**dadurch gekennzeichnet,**
daß man die abgetöteten Targetzellen mit einem DNA-Farbstoff, insbesondere einem Fluoreszenzfarbstoff markiert.

9. Verfahren nach Anspruch 8,
**dadurch gekennzeichnet,**
daß man als DNA-Farbstoff Propidiumjodid verwendet.

10. Reagenzienkit, insbesondere zur Bestimmung der Aktivität von natürlichen Killerzellen (NK-Zellen),
**dadurch gekennzeichnet,**
daß es mit einem Membranfarbstoff markierte kryokonservierte Targetzellen mit einer Vitalität von mindestens 90% nach dem Auftauen und einen DNA-Farbstoff physikalisch voneinander getrennt enthält.

## Claims

1. Method for the determination of the action of effectors on target cells,
**wherein**
thawed cryopreserved mammalian cells are used as target cells without prior culture which have a vitality of at least 90 % after storage at -70°C for a period of 4 weeks and are obtainable by preparing a suspension of target cells in a medium, labelling the target cells, culturing the labelled cells for 4-6 h, adding a cryopreservative up to a final concentration of 2-10 % (v/v) and freezing the cells with a vitality of > 98 % with a temperature decrease of not more than 2°C per minute.

2. Method as claimed in claim 1,
**wherein**
cryopreserved cells are used which have a vitality of at least 90 % after storage at -70°C for a period of 12 weeks.

3. Method as claimed in claim 1 or 2,
**wherein**
a human tumour or leukaemia cell line is used as the target cells.

4. Method as claimed in claim 3,
**wherein**
a human cell line is used which is selected from the chronic myelogenic leukaemia cell line K562 (ATCC CCL 243), the acute lymphoblastic leukaemia T cell line MOLT-4, the acute promyelocytic leukaemia cell line HL-60, the ovarian carcinoma cell line Goodwin and the alveolar cell carcinoma cell line A 549.

5. Method as claimed in one of the claims 1 to 4,
**wherein**
the activity of natural killer cells (NK cells) is determined and the target cells are labelled with a first marker substance before the cryopreservation, mixed and incubated with a sample containing NK cells after thawing and without prior culture, the killed target cells are specifically labelled with a second marker substance and the marker is determined.

6. Method as claimed in one of the claims 1 to 5,
**wherein**
the target cells are labelled with a membrane dye in particular with a lipophilic fluorescent dye.

7. Method as claimed in claim 16,
**wherein**
3,3'-dioctadecyloxacarbocyanine or a salt thereof is used as the membrane dye.

8. Method as claimed in one of the claims 5 to 7,
**wherein**
the killed target cells are labelled with a DNA dye, in particular a fluorescent dye.

9. Method as claimed in claim 8,
**wherein**
propidium iodide is used as the DNA dye.

10. Reagent kit in particular for the determination of the activity of natural killer cells (NK cells),
**wherein**
it contains cryopreserved target cells, a membrane dye and a DNA dye physically separated from one another.

## Revendications

1. Procédé pour déterminer l'action des effecteurs sur des cellules cibles,
caractérisé en ce que l'on utilise comme cellules cibles des cellules de mammifère cryoconservées décongelées, qui après un stockage à -70°C pendant une durée de 4 semaines possèdent une vitalité d'au moins 90% et peuvent être obtenues par préparation d'une suspension de cellules cibles dans un milieu, marquage des cellules cibles, mise en culture des cellules marquées pendant 4-6 heures, addition d'un agent de cryoconservation jusqu'à une concentration finale de 2-10% (volume/volume) et congélation des cellules ayant une vitalité > 98% avec une chute de température ne dépassant pas plus de 2°C par minute.

2. Procédé selon la revendication 1,
caractérisé en ce que l'on utilise des cellules cryoconservées qui, après stockage à -70°C, possèdent pendant une durée de 12 semaines une vitalité d'au moins 90%.

3. Procédé selon la revendication 1 ou 2,
caractérisé en ce que l'on utilise comme cellules cibles une lignée de cellules tumorales ou leucémiques humaines.

4. Procédé selon la revendication 3,
caractérisé en ce que l'on utilise une lignée cellulaire humaine qui est choisie à partir de la lignée cellulaire de la leucémie myélogène chronique K562 (ATCC CCL 243), de la lignée des cellules T de la leucémie lymphoblastique, aiguë MOLT-4, de la lignée cellulaire de la leucémie promyélocytère aiguë HL-60, de la lignée cellulaire du carcinome ovarien Goodwin et de la lignée cellulaire du carcinome alvéocellulaire A 549.

5. Procédé selon l'une des revendications 1 à 4,
caractérisé en ce que l'on détermine l'activité de cellules tueuses naturelles (cellules NK) et l'on marque les cellules cibles avant la cryoconservation avec une première substance de marquage, après la décongélation sans culture préalable, on mélange et on incube l'échantillon contenant une cellule NK, on marque les cellules cibles tuées avec une deuxième substance de marquage de façon spécifique et l'on détermine le marquage.

6. Procédé selon l'une des revendications 1 à 5,
caractérisé en ce que l'on marque les cellules cibles avec un colorant membranaire, en particulier avec un colorant fluorescent lipophile.

7. Procédé selon la revendication 6,
caractérisé en ce qu'en tant que colorant membranaire on utilise une 3,3'-dioctadécyloxacarbocyanine ou son sel.

8. Procédé selon l'une des revendications 5 à 7,
caractérisé en ce que l'on marque les cellules cibles tuées avec un colorant d'ADN, en particulier un colorant fluorescent.

9. Procédé selon la revendication 8,
caractérisé en ce qu'en tant que colorant d'ADN, on utilise l'iodure de propidium.

10. Kit de réactif, en particulier pour la détermination de l'activité de cellules tueuses naturelles (cellules NK), caractérisé en ce qu'il contient des cellules cibles cryoconservées marquées avec un colorant membranaire ayant une vitalité d'au moins 90% après la décongélation et un colorant d'ADN, séparés physiquement entre eux.
